# EUROPEAN PATENT SPECIFICATION

(11) **EP 0 507 622 B1**
(45) Date of publication and mention of the grant of the patent: **17.09.1997**
(21) Application number: 92302984.7
(22) Date of filing: 03.04.1992
(51) Int. Cl.: A61B 17/28, A61B 17/32

(54) **Endoscopic surgical instruments**
Endoskopische chirurgische Instrumente
Instruments chirurgicaux endoscopiques

(30) Priority: 04.04.1991 US 680392; 04.04.1991 US 680399
(43) Date of publication of application: 07.10.1992
(73) Proprietor: SYMBIOSIS CORPORATION, Miami, Florida 33166 (US)
(72) Inventor: Smith, Kevin W., Florida FL-33156 (US); Slater, Charles R., Fort Lauderdale, FL 33312 (US); Murphy, Gregory J., Sunrise, FL 33323 (US); Bales, Thomas O., Miami, FL 33133 (US); Box, John William, Miami, FL 33156 (US); Scarfone, Frank A., Boca Raton, FL 33434 (US)
(74) Representative: VOSSIUS & PARTNER

(56) References cited:
- WO-A-89/11827
- WO-A-91/16856
- US-A- 3 895 636
- US-A- 4 369 788

## Description

The present invention broadly relates to endoscopic surgical instruments. More particularly, the invention relates to surgical instruments which include end effectors such as cutters, graspers, and dissectors which are useful in endoscopy and laparoscopy procedures and which may be disposed of after a single use.

The endoscopy and laparoscopy procedures have recently become widely practiced surgical procedures. The endoscopy and laparoscopy procedures involve incising through body walls (e.g., such as the abdominal wall) for examining, viewing and/or operating on the ovaries, uterus, gall bladder, bowels, appendix, etc. or for general abdominal surgery. Typically, trocars are utilized for creating the incisions. Trocar tubes are left in place in the abdominal wall so that the endoscopic or laparoscopic surgical tools may be inserted through the tube. A camera or magnifying lens is often inserted through the largest diameter trocar tube (e.g. 10mm diameter) which for the laparoscopy procedure is generally located at the navel incision, while a cutter, dissector, or other surgical instrument is inserted through a smaller diameter trocar tube (e.g. 5 mm diameter) for purposes of manipulating and/or cutting the internal organ. Sometimes it is desirable to have several trocar tubes in place at once in order to receive several surgical instruments. In this manner, organ or tissue may be grasped with one surgical instrument, and simultaneously may be cut or stitched with another surgical instrument; all under view of the surgeon via the camera in place in the navel trocar tube.

The endoscopic and laparoscopic tools of the prior art are primarily reusable stainless steel tools. Between each use of a stainless steel tool, the tool must be soaked, scrubbed, and disinfected. The usual procedure is then to dry the tool, wrap it, and put it in a steam autoclave. The tool is kept sterile until just prior to use when it is removed from the autoclave and unwrapped in the locale of the sterile field of use.

While reusable laparoscopic tools have functioned well for their intended purpose, the process of sterilizing the tool is problematic. Small pieces of tissue or organ often become lodged in the end effectors, and much labor is required to ensure that complete sterility is obtained and maintained. In addition, over time, sharp laparoscopic instruments such as a scissors get dull and must be discarded. However, prior to use of a particular instrument, the surgeon is not able to discern the state of the instrument and whether the instrument will satisfy the surgeon's requirements.

WO 89/11827 (Nierman) discloses biopsy forceps for use in a flexible fibreoptic bronchoscope. The device comprises a flexible catheter and a forcep cup assembly attached to the distal end of the catheter by hinges.

US 4369788 (Goald) discloses forceps of the 'alligator jaw' type for use in microlumbar discectomy. Three linkage devices are provided for resisting twisting forces that tend to separate the sliding arms during removal of gristle.

### SUMMARY OF THE INVENTION

It is therefore an object of the invention to provide a disposable endoscopic surgical instrument.

This object is achieved with a surgical instrument according to the claims.

It is an advantage of the invention to provide single and double-acting laparoscopic surgical instruments which utilize improved linkage systems.

In accord with the object of the invention, a surgical instrument for insertion through a trocar tube is provided and generally includes, a tube, a push rod which extends through the tube, an actuating means engaging the tube and the push rod for imparting reciprocal axial motion to the push rod, end effector means coupled to the push rod by linkage means which are also coupled to the push rod, and a clevis coupled to the tube at its proximal end and to the end effector means at its distal end, wherein axial movement of the push rod effects movement of the end effector means in a plane parallel to the longitudinal axis of the push rod. Plastic shrink wrap is preferably utilized to electrically insulate the disposable instrument and extends over the aluminum tube and over at least an adjacent portion of the clevis. The tube and push rod are preferably made of aluminum, the clevis is preferably made of a high-strength aluminum alloy, the actuating means is preferably made of plastic and aluminum, and the end effector means is preferably made of investment cast bronze.

The clevis of the invention is preferably a separately formed clevis having a knurled rod-like proximal end for mating with the end of the aluminum tube, and a post-supporting U-shaped distal portion for holding the end effector means. The post in the distal portion is perpendicular to the legs of the U-shaped distal portion and is arranged to extend through hole(s) in the end effector means. In this manner, the blades or prongs of the end effector means are held by, but can rotate around the post. Each leg of the U-shaped distal portion of the clevis also preferably includes a notch which serves as a terminating location for the shrink-wrap. Another aspect of the clevis relates to the forming of the post integral with one of the legs of the distal portion of the clevis.

The end effector means of the invention can take any of many forms, such as, e.g., a scissors, a dissector, or a grasper. Additionally, the end effector means can be double acting or single acting. Regardless of the type of end effector utilized, the end effector is arranged with a hole to accept the post of the clevis so that the end effector can rotate around the post.

According to one aspect of the invention the push rod is flattened on its distal end, and the linkage means which couples the push rod and the end effector is a staple which extends through a hole in the flattened end of the push rod as well as another hole in the proximal end of the end effector. Because the outer tube is positioned at a fixed distance from the rotation hole in the end effector (due to the clevis), when the push rod is moved axially relative to the tube, the end effector cannot move axially. However, because the push rod is also a fixed distance away from another hole in the proximal end of the end effector (due to the staple), movement of the push rod relative to the tube causes rotation of the end effector in a plane. In other words, movement of the push rod relative to the tube causes the hole through the end effector through which the staple extends to rotate along an arc centered at the rotation hole in the end effector through which the post of the clevis extends. Movement in this manner typically effects a cutting, dissecting or grasping action.

Single acting end effectors can include one hole in the flattened end of the push rod and one staple for connecting the moving prong or blade of the end effector to the push rod A double acting end effector correspondingly includes two holes in the flattened end of the push rod and two staples; one for each prong or blade.

A better understanding of the disposable laparoscopic surgical instruments of the invention, and additional advantages and objects of the invention will become apparent to those skilled in the art upon reference to the detailed description and accompanying drawings.

### BRIEF DESCRIPTION OF THE DRAWINGS

Figure 1 is a side elevation view, partly in section, of the endoscopic instrument prior to insertion into a trocar tube, and, in partial phantom format, after insertion into a trocar tube;
Figure 2a is a side elevation view, partly in section, of the clevis of the invention in conjunction with the distal end of the tube and shrink wrap of the invention;
Figure 2b is a cross-section view of the device of Figure 2a;
Figure 3a is a partially broken-away side elevation view of the actuating handle of the disposable laparoscopic instrument of the invention;
Figure 3b is a rear elevation view of the device of Figure 3a;
Figure 4a is a side elevation view, partly in section, of a double acting dissector in conjunction with the clevis and the distal ends of the rod and tube of the endoscopic instrument of the invention, with the staple linkage means shown in perspective and broken out views;
Figure 4b is a plan view of the device of Figure 4a;
Figure 4c is an enlarged side view of the connection of the distal end of the rod and the proximal ends of the staple linkage means of Fig. 4a;
Figures 4d and 4e are enlarged top views of the distal end of the rod and the proximal ends of the staple linkage means when the blades of the the double acting dissector are in open and closed positions respectively; Figure 4f is a side elevation view of a double acting dissector utilizing a crossed staple linkage means;
Figure 4g is an enlarged top view of the distal end of the rod and the proximal end of the crossed staple linkage means; and
Figure 5 is a side elevation view of a first embodiment of a single acting scissors of the invention in conjunction with the clevis, and the distal ends of the rod and tube of the disposable laparoscopic instrument of the invention.

### DETAILED DESCRIPTION OF THE PREFERRED EMBODIMENT

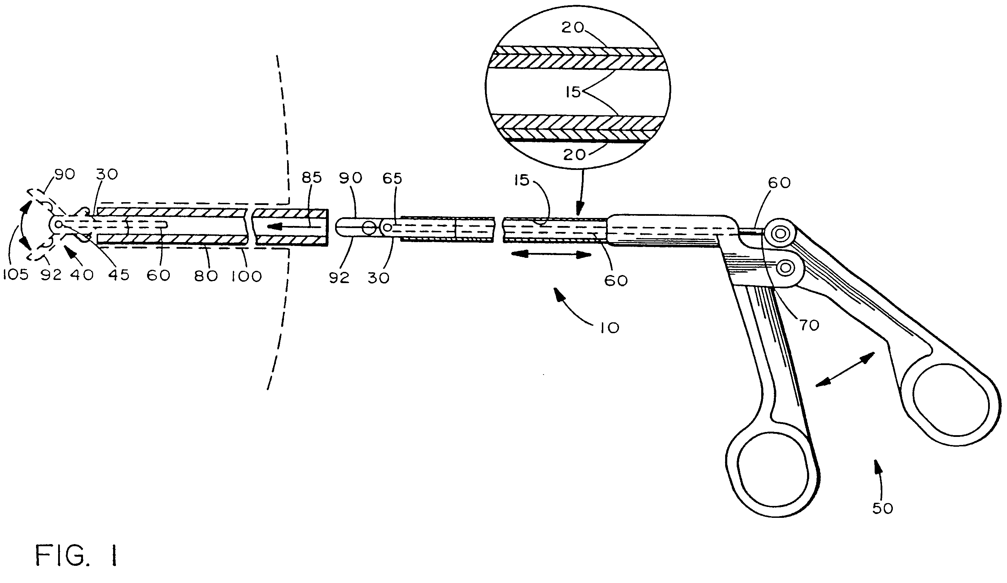

With reference to Figure 1, a disposable endoscopic surgical instrument is indicated at 10. The disposable instrument 10 broadly comprises an aluminum tube 15 surrounded by a peripheral insulating shrink wrap layer of plastic 20, a clevis means 30, end effectors 40, actuating means 50, and a push rod 60. The clevis means 30 is advantageously a separately formed aluminum piece which fixedly engages aluminum tube 15 as described in more detail hereinafter. The clevis 30 also engages the end effectors 40 which are pivotally engaged to clevis 30 at pivot pin 45. The end effectors 40 are preferably formed of investment cast bronze. The push rod 60, which is also formed of aluminum, is engaged at its distal end 65 to the end effectors 40, as hereinafter more fully described, and is connected at 70, at its proximal end to a manually operable actuating means 50. For purposes herein, the "distal end" of the instrument 10 or any part thereof, is the end closest to the surgical site and distant from the surgeon, while the "proximal end" of the instrument 10 or any part thereof, is the end most proximate the surgeon and distant the surgical site.

In use, the endoscopic/laparoscopic instrument 10 is inserted with the blades or graspers 90, 92 of the end effector 40 in the closed position, into trocar tube 80, as indicated at the arrow 85 of Figure 1. The distal portion of the instrument 10 passes through the trocar tube 80 into body incision 100. Upon the distal portion of the instrument 10 exiting the trocar tube 80, the blades 90, 92 can be opened and closed as indicated at 105 by reciprocal motion of push rod 60 which results from operation of the manual actuating means 50. As is discussed more fully hereinafter, the clevis effectively translates the reciprocal motion of the push rod 60 into the end effector means action indicated at 105.

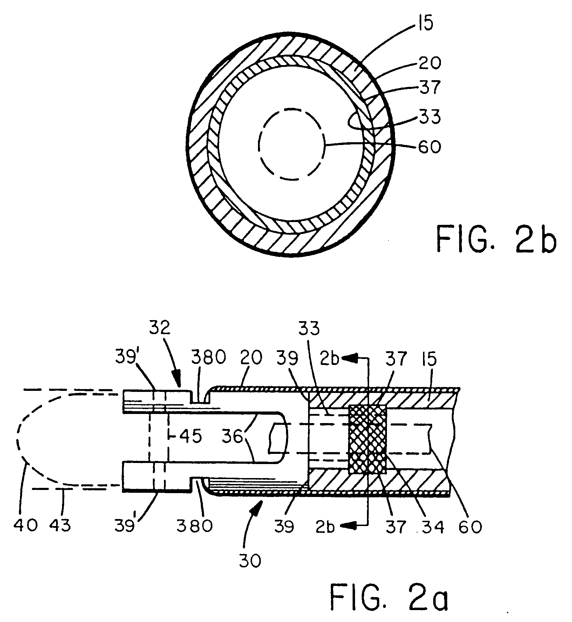

Turning to Figures 2a and 2b, a preferred configuration of the clevis 30 of the present invention is seen. The clevis has a knurled rod-like proximal portion 34 for mating with the end of the aluminum tube 15, and a post-supporting U-shaped distal portion 32 for holding the end effector means. The outer diameter of the distal portion 32 of the clevis is larger than the outer diameter of the proximal portion 34; shoulder 39 being formed therebetween. The proximal portion 34 of the clevis is preferably hollow, as indicated at 33, to permit the push rod 60 to extend therethrough. The distal portion 32 of the clevis 30 is provided with legs 36 and a post or pivot pin 45. The post 45 is generally perpendicular, i.e. transverse, to the legs 36 of the clevis and is arranged to extend through hole(s) 39 in the end effector means 40. In this manner, the blades or prongs of the end effector means 40 are held by, but can rotate around, i.e. are rotatably engaged with the post 45.

As seen in Figure 2a, a recess or notch 380 is provided which extends across each leg 36 of the clevis 30. Consequently, a peripherally applied electrically insulating plastic wrap 20 can be end-cut at recess 380 and a smooth transition from the end effector means 40 via the clevis 30 to tube 15 can be achieved. Even if slight outward flaring of wrap 20 occurs at the end-cut, as is common, this flaring can be tolerated as it will be within the envelope of the normal outer surface indicated at 43.

Clevis 30 is preferably made from a high strength aluminum base alloy (e.g. 2024 alloy of Alcoa) which is preferably harder than the aluminum base alloy (e.,g. 6061 or 6063 alloys of Alcoa) from which tube 15 is fabricated. The post portion of the clevis may be made out of the identical alloy or, for added strength, out of a stainless steel nail. In assembly of the laparoscopy surgical instrument 10, serrated or knurled portion 34 of clevis 10 is fit snugly into tube 15 such that the walls of tube 15 abut the peripheral shoulder 39 of clevis 30, with the outer surface of tube 15 and the adjacent outer surface of clevis 30 having essentially the same diameter. Mechanical pressure is then applied to tube 15 peripherally at the location of knurled portion 34, thereby crimping the end portion of tube 15 onto the knurled portion 34. Mechanical pressure causes the projections of the knurls to bite into and firmly engage tube 15 as indicated at 37 due to the higher hardness of the clevis material. Once the clevis 30 and tube 15 have been properly joined, the plastic shrink wrap 20 can be applied over the tube 15 and an adjacent portion of the clevis 30 and end-cut at recess 380.

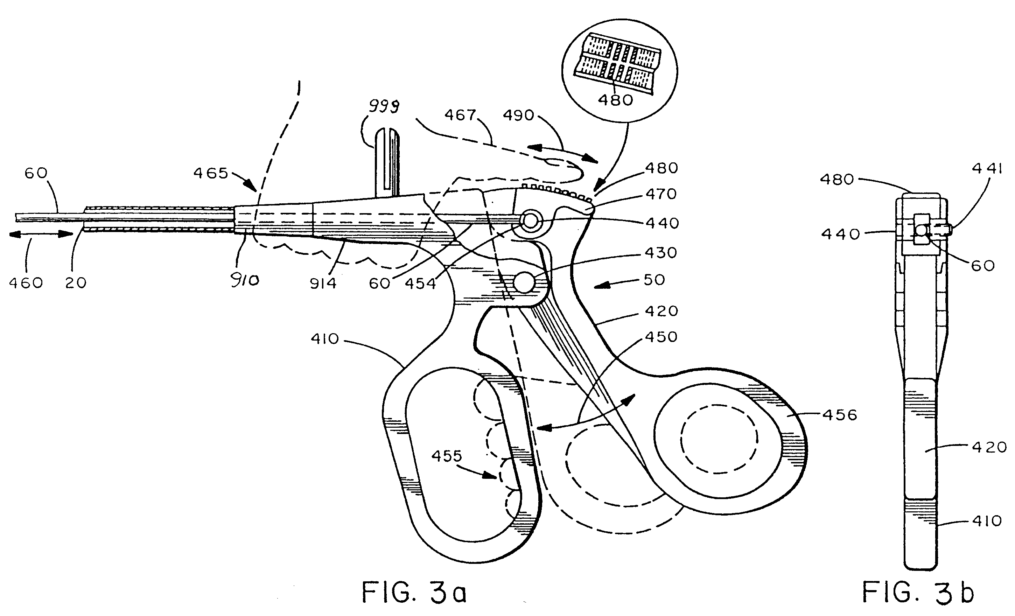

With reference to Figures 3a and 3b, manually operable actuating means are indicated at 50 which includes an electrically insulating housing 914 having a fixed handle portion 410 integral therewith and a lever portion 420 pivotally engaged to housing 914 at pivot pin 430. Push rod 60 passes through aluminum tube 15 (covered by shrink wrap 20) and engages cross pin 440 at 454; set screw 441 being used to extend into cross pin 440 and set push rod 60 in the cross pin 440. The cross pin 440 is fixedly positioned in lever member 420. Upon pivotal motion of lever arm 420, as indicated at 450, using a conventional hand grip as indicated at 455 to apply pressure to extended handle element 456 of lever member 420, push rod 60 will move linearly as indicated at 460 to actuate an end-effector (not shown in Figure 3a) coupled thereto as hereinabove described. There may be occasions, in the course of certain procedures, that certain surgeons will prefer to hold the actuating means 50 in the manner indicated at 465 with fingers grasping housing 914 and the thumb 467 adjacent a portion 470 of lever member 420 which is positioned on the opposite side of cross-pin 440 from extended handle element 456. Thus, in accord with one aspect of the invention, a roughened knurled or serrated surface 480 is provided integral with portion 470 of lever member 420 to enable a frictional engagement with thumb 467. Utilizing serrated surface 480, when thumb motion as indicated at 490 is initiated, pivotal motion of lever arm 420 is accomplished, as indicated at 450, as is the linear motion of push rod 60 as indicated at 460.

Also seen in Fig. 3a is an insulating ferrule 910 which is designed to extend over and bridge the shrink wrap tubing 20 and the housing 914 so as to guarantee that all portions of the tube 15 is insulated. The ferrule 910 permits a cautery procedure to be accomplished (see cautery terminal 999) without concern of shock to the surgeon. Also, preferably, the ferrule 910 is formed of plastic and is color coded so that a different color is used for each instrument or class of instruments. Thus, for example, scissors may be provided with a red ferrule, while graspers might be provided with a yellow ferrule; clamps with a green ferrule; etc.

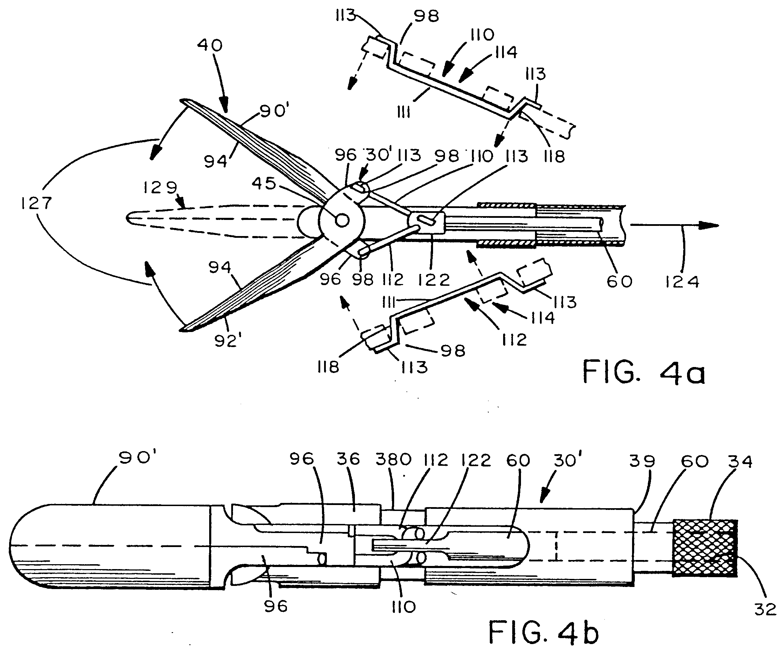

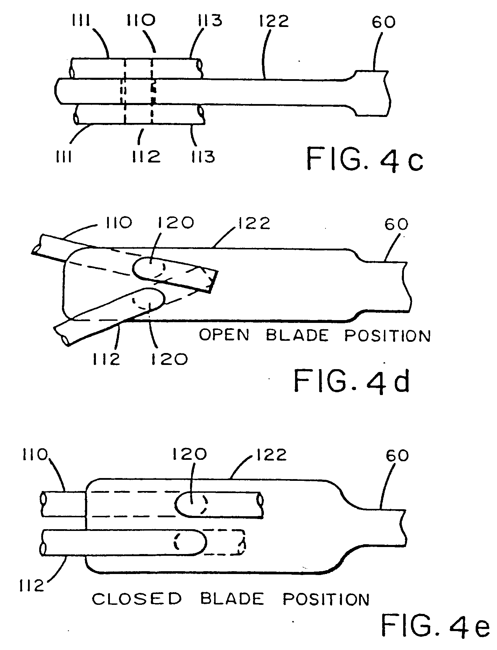

With reference to Figures 4a-4g, details are seen of an end effector 40 and the linkage means for linking the end effector 40 to the push rod 60. In particular, in Figs. 4a-4g, a double acting dissector is shown with blades 90′, 92′ which are respectively rotatably mounted on pivot pin 45 of clevis 80′. Each blade 90′, 92′ of the dissector has a forwardly extending manipulating portion 94, and a rearwardly extending planar base portion 96 with a through-hole 98. Each of the through-holes 98 of planar base portions 96 is separately engaged by a separate connecting or linkage means 110, 112.

As shown in Fig. 4a, according to one preferred embodiment, each linkage means 110, 112 is in the form of a thin metal member generally in the shape of an outwardly flared staple. Each linkage means may be generally described as having a U-shaped section 114 with a base 111 perpendicular to and bridging the arms 118 of the U, and two generally parallel spaced apart outwardly extending side or tab elements 113 which are generally parallel to base 111. Each of the linkage means 110, 112 has one of its tab elements 113 engaged in a through-hole 98 of a planar base 96, with the U-shaped section of the linkage means extending respectively in opposite directions as illustrated. The other tab element 113 of the linkage means 110, 112 engage through-holes 120 formed in a flattened plate-like terminal portion 122 of push rod 60 (as seen more clearly in Figs. 4d-4f). As can be seen from Fig. 4a, movement of push rod 60 in the direction indicated at 124 will cause blades 90′, 92′ to move in the direction indicated at 127 to the position 129 without interference between the oppositely positioned staple-like linkage means 110, 112. Correspondingly, the tab elements 113 of the linkage means 110, 112 which extend through the flattened terminal portion 122 of push rod 60 will move from their position shown in Fig. 4d, to the position shown in Fig. 4e.

That manipulators (blades) 90′ and 92′ open and close in response to the axial movement of push rod 60 may be understood by understanding the relationship of the clevis 30′ and linkage means 110, 112 to the blades 90′, 92′, the push rod 60, and the tube 15. In particular, due to the fact that the clevis 30′ is rigidly attached to the tube 15 (as described above with reference to Fig. 2a), the tube 15 is a fixed distance from the rotation pin 45 of the clevis, and hence to the holes in the blades 90′ and 92′ through which rotation pin 45 extends. Thus, when the push rod 60 is moved axially relative to the tube 15 (the tube being fixed in place), the blades of the end effector cannot move axially with the push rod. However, because the push rod 60 is also a fixed distance away from holes 98 in the base portion of the end effector blades (due to staple linkage means 110, 112), movement of the push rod relative to the tube must cause movement of the holes 98 in the end effector blades. Because one part each blade is fixed, but another part must move when the push rod 60 is moved relative to the tube 15, end effector blades 110 and 112 rotate along an arc centered at the fixed rotation hole in the end effector through which the post 45 of the clevis 30′ extends. Movement in this manner typically effects a cutting or grasping action.

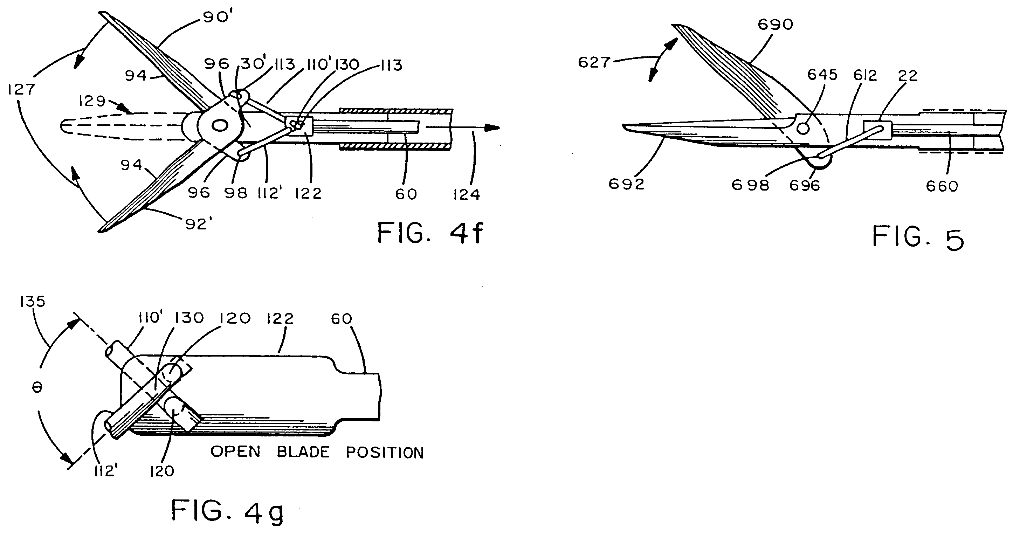

Figures 4f and 4g show another embodiment of the linkage means which increases the stability of the end effector means 40. In Figures 4g and 4h it is seen that the linkage means 110′, 112′ do not respectively engage the nearest of the through-holes 120 transversely aligned in the plate-like terminal portion 122 of push rod 60, but instead cross-over as indicated at 130. With this arrangement the angle θ, indicated at 135, is increased. The increase in this angle affords a more stable instrument, because the amount of "shake" which results from the unavoidable clearances at the pivot 45 is reduced; i.e., as θ approaches 90°, the amount of free movement at the end of an end-effector blade 90′, 92′ is minimized.

Figure 5 shows a single acting scissors. Essentially, the single acting scissors is identical to the double acting scissors of Figs. 4a-4g except that blade 692 is stationary; hence no staple is used to connect blade 692 (which may be intregal with the clevis) to rod 660. While blade 692 is stationary, blade 690 pivots as indicated at 627 around pin 645. To ensure rotational movement of blade 690 upon axial movement of rod 660, the end 22 of rod 660 should be supported.

The preferred endoscopic/laparoscopic instruments of the invention are preferably assembled in the following fashion. The knurled portion 34 of the clevis 30 of the invention is inserted into the aluminum tube 15 which had been previously insert molded in the fixed handle portion 914, 410 of the actuating means 50. The aluminum tube 15 is crimped over the knurls 37 to effect mating. Shrink wrap 20 is then applied over the aluminum tube 15 and end-cut at grooves 380 in the arms 36 of the clevis 30. Ferrule 910 is slid over the distal end of the aluminum tube 15, up over the end of the housing 914, and snapped into place, thereby providing complete insulation. The rod 60, staples 110, 112, and end effectors (e.g. 90, 92) are assembled, with the staples coupling the rod to the end effectors. The rod is slid through the clevis and down the aluminum tube, until the end effectors are located between the arms of the clevis. When the holes in the proximal end of the end effectors (e.g. 96, 98) are lined up with the through-holes 39 in the arms of the clevis, the rotation post 45, which may either be integral with the clevis, or a separate post or nail, is inserted through the holes in the end effectors, and secured in the holes of the clevis arms such as by tapping. At this point, all that remains to be assembled is the actuating means 50. To assemble the actuating means, a cross pin 440 is inserted in handle 420. Handle 420 is then arranged such that the push rod 60 which extends out of the fixed handle portion will extend through the cross pin 440. With rod 60 in the cross pin 440, handle 420 is lined up with handle 410 such that the handle rotation pivot pin 430 can be inserted. With pivot pin 430 in place, and with the end effectors in the closed position, set screw 441 is tightened into the cross pin until it bites into rod 60, thereby holding rod 60 in place relative to cross pin 440.

There has been described and illustrated herein endoscopic instruments. While particular embodiments of the invention have been described, it is not intended that the invention be limited exactly thereto, as it is intended that the invention be as broad in scope as the art will permit. Thus, while particular end effectors were disclosed, it will be appreciated that other end effectors such as, e.g., duckbill graspers, duck-bill dissectors, atraumatic graspers, and traumatic (rat-tooth) graspers, etc., could be utilized. Also, while various materials were described as being preferred for various parts, it will be appreciated that other materials could be utilized. By way of example only, and not by way of limitation, while the tube and clevis are preferably made from aluminum alloys, with the clevis being harder than the tube, if desired, the tube could be harder than the clevis. In such a situation, rather than crimping the tube over the clevis, the clevis could be welded or press fit into the tube. Therefore, it will be apparent to those skilled in the art that other changes and modifications may be made to the invention as described in the specification without departing from the scope of the invention as so claimed.

## Claims

1. A surgical instrument (10) for insertion through a trocar tube, comprising a reciprocally movable push rod (60) having proximal and distal ends, a pivotally rotatable first end effector (40), and a second end effector (40), an actuating means (50) coupled to said push rod (60) at said proximal end of said push rod (60), an outer tube (15) through which said push rod (60) extends and to which said actuation means (50) is coupled, characterised in that said instrument comprises:
coupling means for coupling said push rod (60) and said first end effector (40), said coupling means comprising a unitary resilient staple-like element (110) having
a first terminal portion (113) pivotally engaging a through-hole (120) in the distal portion of said push rod (60),
an oppositely extending second terminal portion (113) pivotally engaging a second through-hole (98) in the first end effector (40), and
a substantially U-shaped middle portion (114) coupling said first and second terminal portions (113).

2. A surgical instrument as claimed in Claim 1 wherein:
said push rod (60) has a longitudinal axis, and said unitary resilient staple-like element (110) is angled relative to said longitudinal axis of said push rod (60).

3. A surgical instrument as claimed in Claim 1 or Claim 2 wherein:
said first (120) and second (98) through-holes are substantially transverse to said longitudinal axis and parallel to each other.

4. A surgical instrument as claimed in any one of the preceding Claims wherein:
said distal end (122) of said push rod (60) is flattened and includes said through-hole (120) in said distal end of said push rod (60).

5. A surgical instrument as claimed in any one of the preceding Claims comprising two pivotally rotatable end effectors (40) and further comprising coupling means for coupling said push rod (60) and said two pivotally rotatable end effectors (40) comprising
a second unitary resilient staple-like element (112) having
a first terminal portion (113) pivotally engaging a second of a pair of through-holes (120) in the push rod (60),
an oppositely extending second terminal portion (113) pivotally engaging a through-hole (98) in a second of said two end effectors (40), and
a substantially U-shaped middle portion (114) coupling said first and second terminal portions (113) of said second unitary resilient staple-like element (112).

6. A surgical instrument as claimed in Claim 5 wherein:
said push rod (60) has a longitudinal axis,
said first of said pair of through-holes (120) in the push rod (60) is located above said second of said pair of through-holes (120) in the push rod (60) relative to said longitudinal axis,
said through-hole (98) in said first of said two end effectors (40) is located below said through-hole (98) in said second of said two end end effectors (40) relative to said longitudinal axis, and
said first terminal portion (113) of said first unitary resilient staple-like element (110) engages the second of said pair of through-holes (120) in the push rod (60) and the first terminal portion (113) of said second unitary resilient staple-like element (112) engages the first of said pair of through-holes (120) in the push rod (60).

7. A surgical instrument as claimed in Claim 5 or Claim 6 wherein:
said first (110) and second (112) unitary resilient staple-like elements are both angled relative to the longitudinal axis of said push rod (60).

8. A surgical instrument as claimed in Claim 6 or Claim 7 wherein:
said pair of through-holes (120) in said push rod (60) and said through-holes (98) in said end effectors (40) are substantially transverse to said longitudinal axis of said push rod (60) and parallel to each other.

9. A surgical instrument (10) as claimed in any one of the preceding Claims wherein:
a) said outer tube is a hollow tube (15) having a first end and a second end;
b) said coupling means further comprises a clevis means (30) separately formed from said tube (15) and mechanically coupled to said first end of said tube (15), said clevis means (30) having first rotation means (45) substantially transverse to said longitudinal axis;
c) two end effectors (40) are provided for grasping, cutting, clamping, dissecting, or extracting, one of said end effector (40) having a first hole (39) for receiving said first rotation means (45) and for pivotally engaging said clevis means (30) at said rotation means (45) and a second hole (98) substantially parallel to said first hole (39);
d) said push rod (60) extends at least partially through said hollow tube (15) and has a first end and a second end, said rod (60) having a third hole (120);
e) a second unitary resilient staple-like element (112) links said first end of said rod (60) with one of said end effectors (40) having said first and second holes, said second unitary resilient staple-like element (112) having a first terminal portion (113) pivotally engaging said third hole (120) of said rod, an oppositely extending second terminal portion (113) pivotally engaging said second hole (98) of said end effector means having said first (39) and second (98) holes, and a substantially U-shaped middle portion (114) coupling said first and second terminal portions (113); and
f) said actuating means (50) is engaged to said second end of said rod (60) for imparting reciprocal motion to said rod (60) relative to said tube (15) which is translated at said clevis means (30) to pivotal motion of said end effector (40) having said first (39) and second (98) holes.

10. A surgical instrument as claimed in Claim 9, wherein:
said hollow tube (15), clevis means (30), and push rod means (60) are formed of aluminium or aluminium base alloys.

11. A surgical instrument as claimed in Claim 9 or Claim 10 further comprising:
g) shrink wrap plastic (20) covering substantially all of said hollow tube (15), and a portion of said clevis means (30) adjacent the first end of the hollow tube (15).

12. A surgical instrument as claimed in Claim 11 wherein:
said hollow tube (15) has uniform inner and outer diameters, and
said clevis means (30) includes a hollow first portion (34) having an inner diameter, and having an outer diameter slightly smaller than said inner diameter of said hollow tube (15), said hollow first portion (34) fitting closely within the hollow tube (15) and fixedly engaging the hollow tube (15), and a second portion (32) having a base section having an outer diameter substantially the same as the outer diameter of the hollow tube (15) and a bore corresponding to the inner diameter of the hollow first portion (34) of the clevis means (30), and a pair of parallel, spaced apart, opposed arm members (36) having respective first and second ends, and integrally joined to said base portion of said clevis means (30) at respective said first ends of said opposed arm members (36), said pair of arm members (36) extending away from said hollow first portion of said clevis means (30), each said opposed arm member (36) having an undercut groove (380) therein along an outer surface of said arm member (36) and substantially transverse said longitudinal axis, wherein said shrink wrap plastic covering (20) terminates in the undercut grooves (380) of the arm members (36).

13. A surgical instrument as claimed in Claim 11 or Claim 12 further comprising:
a ferrule means (910) for surrounding and covering a terminal portion of said shrink wrap plastic covering (20) and an adjacent portion of said actuating means (50).

14. A surgical instrument as claimed in Claim 13, wherein:
said actuating means (50) comprises a housing means (914) fixedly engaging said aluminium tube (15), wherein said ferrule means (910) surrounds and covers at least a portion of said housing means (914), and a lever means (420) fixedly engaging said push rod (60) and pivotally engaging said housing means (914), wherein upon pivotal movement of said lever means (420) reciprocal motion is imparted to said push rod (60).

15. A surgical instrument as claimed in Claim 14, wherein:
said actuating means (50) further comprises a handle means (456) fixedly attached to said housing means (914) to cooperate with said lever means (420) in providing pivotal movement of said lever means (420) where said handle (456) and lever means (420) are squeezed by a human hand.

16. A surgical instrument as claimed in any one of Claims 1 to 12, wherein:
said actuating means (50) comprises a housing means (914) fixedly engaging said outer tube (15), a lever means (420) fixedly engaging said push rod (60) and pivotally engaging said housing means (914), and a handle means (456) fixedly attached to said housing means (914), wherein pivotal movement of said said lever means (420) imparts reciprocal motion to said push rod (60), and wherein said handle means (456) cooperates with said lever means (420) in providing pivotal movement of said lever means (420) where said handle (456) and lever (420) means are squeezed by a human hand.

17. A surgical instrument as claimed in any one of Claims 9 to 16 wherein:
said first end of said rod (60) has a plate-like portion (122) having said third hole (120) and a fourth hole (120) spaced from said third hole (120),
each of said end effectors (40) has a respective first hole (39) for receiving said first rotation means (45) and for pivotally engaging said clevis means (30) at said rotation means (45), and a respective second hole (98) substantially parallel to said first hole, and
said surgical instrument comprises a second unitary resilient staple-like element (112) linking said first end of said rod (60) with one of said end effectors (40), with one of said unitary resilient staple-like connecting means (110) engaging said third hole (120), of said rod, and the other of said unitary resilient staple-like elements (112) engaging said fourth hole (120) of said rod.

18. A surgical instrument as claimed in Claim 17, wherein:
said first and second elements (110, 112) which engage respective through-holes (120) of the plate-like portion (122) of said push rod (60) are positioned to cross each other.

## Patentansprüche

1. Chirurgisches Instrument (10) zum Einführen durch ein Trokarrohr, mit einer hin- und herbeweglichen Schubstange (60) mit einem proximalen und einem distalen Ende, einer drehbaren ersten Endhandhabungseinrichtung (40) und einer zweiten Endhandhabungseinrichtung (40), einer mit der Schubstange (60) am proximalen Ende der Schubstange (60) verbundenen Betätigungseinrichtung (50) und einem Außenrohr (15), durch das sich die Schubstange (60) erstreckt und mit dem die Betätigungseinrichtung (50) verbunden ist, **dadurch gekennzeichnet**, **daß** das Instrument aufweist:
eine Verbindungseinrichtung zum Verbinden der Schubstange (60) und der ersten Endhandhabungseinrichtung (40), wobei die Verbindungseinrichtung ein einstückiges, elastisches klammerähnliches Element (110) aufweist, mit:
einem ersten Endabschnitt (113), der in eine Durchgangsöffnung (120) im distalen Abschnitt der Schubstange (60) drehbar eingreift;
einem sich entgegengesetzt erstreckenden zweiten Endabschnitt (113), der in eine zweite Durchgangsöffnung (98) in der ersten Endhandhabungseinrichtung (40) drehbar eingreift; und
einem im wesentlichen U-förmigen Mittelabschnitt (114), durch den der erste und der zweite Endabschnitt (113) verbunden sind.

2. Chirurgisches Instrument nach Anspruch 1, wobei
die Schubstange (60) eine Längsachse aufweist und das einstückige, elastische klammerähnliche Element (110) bezüglich der Längsachse der Schubstange (60) winklig angeordnet ist.

3. Chirurgisches Instrument nach Anspruch 1 oder 2, wobei
die erste (120) und die zweite (98) Durchgangsöffnung im wesentlichen quer zur Längsachse und parallel zueinander angeordnet sind.

4. Chirurgisches Instrument nach einem der vorangehenden Ansprüche, wobei
das distale Ende (122) der Schubstange (60) abgeflacht ist und im distalen Ende der Schubstange (60) eine Durchgangsöffnung (120) vorgesehen ist.

5. Chirurgisches Instrument nach einem der vorangehenden Ansprüche, mit zwei drehbaren Endhandhabungseinrichtungen (40) und ferner mit einer Verbindungseinrichtung zum Verbinden der Schubstange (60) und der beiden drehbaren Endhandhabungseinrichtungen (40), mit:
einem zweiten einstückigen, elastischen klammerähnlichen Element (112) mit:
einem ersten Endabschnitt (113), der in eine zweite Durchgangsöffnung eines Paars von Durchgangsöffnungen (120) in der Schubstange (60) drehbar eingreift;
einem sich entgegengesetzt erstreckenden zweiten Endabschnitt (113), der in eine zweite Durchgangsöffnung (98) in einer zweiten der beiden Endhandhabungseinrichtungen (40) drehbar eingreift; und
einem im wesentlichen U-förmigen Mittelabschnitt (114), durch den der erste und der zweite Endabschnitt (113) des zweiten einstückigen, elastischen klammerähnlichen Elements (112) verbunden sind.

6. Chirurgisches Instrument nach Anspruch 5, wobei
die Schubstange (60) eine Längsachse aufweist;
die erste Durchgangsöffnung des Paars Durchgangsöffnungen (120) in der Schubstange (60) bezüglich der Längsachse über der zweiten Durchgangsöffnung des Paars Durchgangsöffnungen (120) in der Schubstange (60) angeordnet ist;
die Durchgangsöffnung (98) in der ersten der beiden Endhandhabungseinrichtungen (40) bezüglich der Längsachse unter der Durchgangsöffnung (98) in der zweiten der beiden Endhandhabungseinrichtungen (40) angeordnet ist; und
der erste Endabschnitt (113) des ersten einstückigen, elastischen klammerähnlichen Elements (110) in die zweite Durchgangsöffnung des Paars Durchgangsöffnungen (120) in der Schubstange (60) eingreift und der erste Endabschnitt (113) des zweiten einstückigen, elastischen klammerähnlichen Elements (112) in die erste Durchgangsöffnung des Paars Durchgangsöffnungen (120) in der Schubstange (60) eingreift.

7. Chirurgisches Instrument nach Anspruch 5 oder 6, wobei
sowohl das erste (110) als auch das zweite (112) einstückige, elastische klammerähnliche Element bezüglich der Längsachse der Schubstange (60) winklig angeordnet sind.

8. Chirurgisches Instrument nach Anspruch 6 oder 7, wobei
das Paar Durchgangsöffnungen (120) in der Schubstange (60) und die Durchgangsöffnungen (98) in den Endhandhabungseinrichtungen (40) im wesentlichen quer zur Längsachse der Schubstange (60) und parallel zueinander angeordnet sind.

9. Chirurgisches Instrument (10) nach einem der vorangehenden Ansprüche, wobei:
a) das Außenrohr ein Hohlrohr (15) mit einem ersten Ende und einem zweiten Ende ist;
b) die Verbindungseinrichtung ferner eine separat vom Rohr (15) ausgebildete Bügeleinrichtung (30) aufweist, die mit dem ersten Ende des Rohrs (15) mechanisch verbunden ist, wobei die Bügeleinrichtung (30) eine im wesentlichen quer zur Längsachse ausgerichtete erste Dreheinrichtung (45) aufweist;
c) die beiden Endhandhabungseinrichtungen (40) zum Greifen, Schneiden, Klemmen, Sezieren bzw. Präparieren oder Extrahieren vorgesehen sind, wobei eine der Endhandhabungseinrichtungen (40) aufweist: eine erste Öffnung (39) zum Aufnehmen der ersten Dreheinrichtung (45) und zum drehbaren Halten der Bügeleinrichtung (30) an der Dreheinrichtung (45) und eine im wesentlichen parallel zur ersten Öffnung (39) angeordnete zweite Öffnung (98);
d) die Schubstange (60) sich mindestens teilweise durch das Hohlrohr (15) ersreckt und ein erstes Ende und ein zweites Ende aufweist und die Schubstange (60) eine dritte Öffnung (120) aufweist;
e) ein zweites einstückiges, elastisches klammerähnliches Element (112) das erste Ende der Stange (60) mit einer der Endhandnabungseinrichtungen (40), die die erste und die zweite Öffnung aufweist, verbindet, wobei das zweite einstückige, elastische klammerähnliche Element (112) aufweist: einen ersten Endabschnitt (113), der in die dritte Öffnung (120) der Stange drehbar eingreift, einen entgegengesetzten zweiten Endabschnitt (113), der in die zweite Öffnung (98) der Endhandhabungseinrichtung drehbar eingreift, die die erste (39) und die zweite (98) Öffnung aufweist, und einen im wesentlichen U-förmigen Mittelabschnitt (114), der den ersten und den zweiten Endabschnitt (113) verbindet; und
f) die Betätigungseinrichtung (50) mit dem zweiten Ende der Stange (60) in Eingriff steht, um auf die Stange (60) bezüglich des Rohrs (15) eine hin- und hergehende Bewegung zu übertragen, die an der Bügeleinrichtung (30) in eine Drehbewegung der Endhandhabungseinrichtung (40) umgesetzt wird, die die erste (39) und die zweite (98) Öffnung aufweist.

10. Chirurgisches Instrument nach Anspruch 9, wobei
das Hohlrohr (15), die Bügeleinrichtung (30) und die Schubstange (60) aus Aluminium oder einer auf Aluminium basierenden Legierung hergestellt sind.

11. Chirurgisches Instrument nach Anspruch 9 oder 10, ferner mit:
g) einem schrumpfumhüllenden Kunststoff (20), der im wesentlichen das gesamte Hohlrohr (15) und einen an das erste Ende des Hohlrohrs (15) angrenzenden Teil der Bügeleinrichtung (30) bedeckt.

12. Chirurgisches Instrument nach Anspruch 11, wobei
das Hohlrohr (15) einen konstanten Innen- und einen konstanten Außendurchmesser aufweist; und
die Bügeleinrichtung (30) aufweist: einen ersten Hohlabschnitt (34) mit einem Innendurchmesser und einem Außendurchmesser, der etwas kleiner ist als der Innendurchmesser des Hohlrohrs (15), wobei der erste Hohlabschnitt (34) innerhalb des Hohlrohrs (15) eng eingepaßt ist und mit dem Hohlrohr (15) fest in Eingriff steht, und einen zweiten Abschnitt (32) mit einem Basisabschnitt, dessen Außendurchmesser im wesentlichen dem Außendurchmesser des Hohlrohrs (15) gleich ist, und einer dem Innendurchmesser des ersten Hohlabschnitts (34) der Bügeleinrichtung (30) entsprechenden Öffnung, und ein Paar parallele, voneinander beabstandete, gegenüberliegende Armelemente (36) mit jeweils einem ersten und einem zweiten Ende, wobei die Armelemente mit dem Basisabschnitt der Bügeleinrichtung (30) an den zugeordneten ersten Enden der gegenüberliegenden Armelemente (36) einstückig verbunden sind, wobei sich das Paar Armelemente (36) vom ersten Hohlabschnitt der Bügeleinrichtung (30) weg erstreckt, jedes der gegenüberliegenden Armelemente (36) eine darin entlang einer Außenfläche des Armelements (36) ausgebildete unterschnittene Nut (380) aufweist, die sich im wesentlichen quer zur Längsachse erstreckt, und die schrumpfumhüllende Kunststoffummantelung (20) in den unterschnittenen Nuten (380) der Armelemente (36) endet.

13. Chirurgisches Instrument nach Anspruch 11 oder 12, ferner mit:
einer Muffeneinrichtung (910) zum Umschließen und Abdecken eines Endabschnitts der schrumpfumhüllenden Kusntstoffummantelung (20) und eines angrenzenden Abschnitts der Betätigungseinrichtung (50).

14. Chirurgisches Instrument nach Anspruch 13, wobei
die Betätigungseinrichtung (50) aufweist: eine mit dem Aluminiumrohr (15) fest in Eingriff stehende Gehäuseeinrichtung (914), wobei die Muffeneinrichtung (910) mindestens einen Teil der Gehäuseeinrichtung (914) umschließt und abdeckt, und eine mit der Schubstange (60) fest in Eingriff stehende und in die Gehäuseeinrichtung (914) drehbar eingreifende Hebeleinrichtung (420), wobei die Drehbewegung der Hebeleinrichtung (420) in eine hin- und hergehende Bewegung der Schubstange (60) umgesetzt wird.

15. Chirurgisches Instrument nach Anspruch 14, wobei
die Betätigungseinrichtung (50) außerdem eine an der Gehäuseeinrichtung (914) stabil befestigte Griffeinrichtung (456) aufweist, die mit der Hebeleinrichtung (420) zusammenwirkt, um eine Drehbewegung der Hebeleinrichtung (420) zu erzeugen, wenn die Griffeinrichtung (456) und die Hebeleinrichtung (420) von Hand zusammengedrückt werden.

16. Chirurgisches Instrument nach einem der Ansprüche 1 bis 12, wobei
die Betätigungseinrichtung (50) aufweist: eine mit dem Außenrohr (15) fest in Eingriff stehende Gehäuseeinrichtung (914), eine mit der Schubstange (60) fest in Eingriff stehende Hebeleinrichtung (420), die in die Gehäuseeinrichtung (914) drehbar eingreift, und eine an der Gehäuseeinrichtung (914) stabil befestigte Griffeinrichtung (456), wobei die Drehbewegung der Hebeleinrichtung (420) in eine hin- und hergehende Bewegung der Schubstange (60) umgesetzt wird und die Griffeinrichtung (456) mit der Hebeleinrichtung (420) zusammenwirkt, um eine Drehbewegung der Hebeleinrichtung (420) zu erzeugen, wenn die Griffeinrichtung (456) und die Hebeleinrichtung (420) von Hand zusammengedrückt werden.

17. Chirurgisches Instrument nach einem der Ansprüche 9 bis 16, wobei
das erste Ende der Stange (60) einen plattenähnlichen Abschnitt (122) mit der dritten Öffnung (120) und einer von der dritten Öffnung (120) beabstandeten vierten Öffnung (120) aufweist;
jede der Endhandhabungseinrichtungen (40) jeweils eine erste Öffnung (39) aufweist, die die erste Dreheinrichtung (45) aufnimmt und in die die Bügeleinrichtung (30) an der Dreheinrichtung (45) drehbar eingreift, und jeweils eine im wesentlichen parallel zur ersten Öffnung ausgerichtete zweite Öffnung (98); und
das chirurgische Instrument ein zweites einstückiges, elastisches klammerähnliches Element (112) aufweist, das das erste Ende der Stange (60) mit einer der Endhandhabungseinrichtungen (40) verbindet, wobei eines der einstückigen, elastischen klammerähnlichen Elemente (110) in die dritte Öffnung (120) der Stange eingreift und das andere der einstückigen, elastischen klammerähnlichen Elemente (112) in die vierte Öffnung (120) der Stange eingreift.

18. Chirurgisches Instrument nach Anspruch 17, wobei
das erste und das zweite Element (110, 112), die in entsprechende Durchgangsöffnungen (120) des plattenähnlichen Abschnitts (122) der Schubstange (60) eingreifen, so angeordnet sind, daß sie sich kreuzen.

## Revendications

1. Instrument chirurgical (10) pour l'introduction à travers un trocart, comprenant une tige de poussée mobile en mouvement de va-et-vient (60) avec des extrémités proxmale et distale, un premier effecteur (40) d'extrémité rotatif de façon pivotante, et un second effecteur d'extrémité (40), un moyen d'actionnement (50) accouplé sur la tige de poussée (60) sur l'extrémité proximale de la tige de poussée (60), un tube extérieur (15) à travers lequel s'étend la tige de poussée (60) et à laquelle est accouplé le moyen d'actionnement (50), caractérisé en ce que l'instrument comprend :
des moyens d'accouplement destinés à accoupler la tige de poussée (60) et le premier effecteur d'extrémité (40), les moyens d'accouplement comprenant un élément en forme d'agrafe élastique unitaire (110) avec
une première portion terminale (113), s'engageant de façon pivotante dans un trou traversant (120) dans la portion distale de la tige de poussée (60),
une seconde portion terminale (113) s'étendant en regard, s'engageant de façon pivotante dans un second trou traversant (98) dans le premier effecteur d'extrémité (40), et
une portion médiane sensiblement en forme de U (114) accouplant les première et seconde portions terminales (113).

2. Instrument chirurgical selon la revendication 1, dans lequel :
la tige de poussée (60) présente un axe longitudinal et l'élément en forme d'agrafe élastique unitaire (110) forme un angle par rapport à l'axe longitudinal de la tige de poussée (60).

3. Instrument chirurgical selon la revendication 1 ou la revendication 2, dans lequel :
les premier (120) et second (98) trous traversants sont sensiblement transversaux par rapport à l'axe longitudinal et sont parallèles entre eux.

4. Instrument chirurgical selon l'une quelconque des revendications précédentes, dans lequel :
l'extrémité distale (122) de la tige de poussée (60) est aplatie et comprend le trou traversant (120) dans l'extrémité distale de la tige de poussée (60).

5. Instrument chirurgical selon l'une quelconque des revendications précédentes, comprenant deux effecteurs d'extrémité (40) rotatifs de façon pivotante et comprenant de plus des moyens d'accouplement permettant d'accoupler la tige de poussée (60) et les deux effecteurs d'extrémité (40) rotatifs de façon pivotante, comprenant
un second élément en forme d'agrafe élastique unitaire (112) avec
une première portion terminale (113) s'engageant de façon pivotante dans le second de la paire de trous traversants (120) dans la tige de poussée (60),
une seconde portion terminale (113) s'étendant en regard, s'engageant de façon pivotante dans un second trou traversant (98) dans le second des deux effecteurs d'extrémité (40), et
une portion médiane sensiblement en forme de U (114) accouplant les première et seconde portions terminales (113) du second élément en forme d'agrafe élastique unitaire (112).

6. Instrument chirurgical selon la revendication 5, dans lequel :
la tige de poussée (60) présente un axe longitudinal,
le premier de la paire de trous traversants (120) dans la tige de poussée (60) est situé au-dessus du second de la paire des trous traversants (120) dans la tige de poussée (60) par rapport à l'axe longitudinal,
le trou traversant (98) dans le premier des deux effecteurs d'extrémité (40) est situé au-dessous du trou traversant (98) dans le second des deux effecteurs d'extrémité (40) par rapport à l'axe longitudinal, et
la première portion terminale (113) du premier élément en forme d'agrafe élastique unitaire (110) s'engage dans le second de la paire de trous traversants (120) dans la tige de poussée (60) et la première portion terminale (113) du second élément en forme d'agrafe élastique unitaire (112) s'engage dans le premier de la paire de trous traversants (120) dans la tige de poussée (60).

7. Instrument chirurgical selon la revendication 5 ou la revendication 6, dans lequel :
les premier (110) et second (112) éléments en forme d'agrafe élastique unitaire présentent tous deux un angle par rapport à l'axe longitudinal de la tige de poussée (60).

8. Instrument chirurgical selon la revendication 6 ou la revendication 7, dans lequel :
la paire de trous traversants (120) dans la tige de poussée (60) et les trous traversants (98) dans les effecteurs d'extrémité (40) sont sensiblement transversaux à l'axe longitudinal de la tige de poussée (60) et parallèles entre eux.

9. Instrument chirurgical (10) selon l'une quelconque des revendications précédentes, dans lequel
a) le tube extérieur est un tube creux (15) avec une première extrémité et une seconde extrémité ;
b) les moyens d'accouplement comprennent de plus une manille (30) formée séparément du tube (15) et accouplée mécaniquement à la première extrémité du tube (15), cette manille (30) comportant des premiers moyens de rotation (45) sensiblement transversaux à l'axe longitudinal ;
c) deux effecteurs d'extrémité (40) sont prévus pour les opérations de préhension, coupe, serrage, dissection ou extraction, l'un de ces effecteurs d'extrémité (40) ayant un premier trou (39) pour recevoir le premier moyen de rotation (45) et pour s'engager de façon pivotante dans la manille (30) au niveau du moyen de rotation (45) et un second trou (98) sensiblement parallèle au premier trou (39);
d) la tige de poussée (60) s'étend au moins partiellement à travers le tube creux (15) et présente une première extrémité et une seconde extrémité, la tige (60) ayant un troisième trou (120) ;
e) un second élément en forme d'agrafe élastique unitaire (112) relie la première extrémité de la tige (60) à l'un des effecteurs d'extrémité (40) ayant le premier et le second trou, le second élément en forme d'agrafe élastique unitaire (112) ayant une première portion terminale (113) s'engageant de façon pivotante dans le troisième trou (120) de la tige, une seconde portion terminale (113) s'étendant en regard, s'engageant par pivotement dans le second trou (98) de l'effecteur d'extrémité comportant les premier (39) et second (98) trous, et une portion médiane sensiblement en forme de U (114) accouplant les première et seconde portions terminales (113) ; et
f) le moyen d'actionnement (50) s'engage dans la seconde extrémité de la tige (60) pour transmettre un mouvement de va-et-vient à la tige (60) par rapport au tube (15) qui est translaté au niveau de la manille (30) en mouvement pivotant de l'effecteur d'extrémité (40) comportant les premier (39) et second (98) trous.

10. Instrument chirurgical selon la revendication 9, dans lequel :
le tube creux (15), la manille (30) et la tige de poussée (60) sont réalisés en aluminium ou en alliage à base d'aluminium.

11. Instrument chirurgical selon la revendication 9 ou la revendication 10, comprenant de plus :
g) un plastique thermorétractable (20) recouvrant sensiblement la totalité du tube creux (15) et une portion de la manille (30) contiguë à la première extrémité du tube creux (15).

12. Instrument chirurgical selon la revendication 11, dans lequel :
le tube creux (15) présente des diamètres interne et externe uniformes, et
la manille (30) comprend une première portion creuse (34) avec un diamètre interne et ayant un diamètre externe légèrement inférieur au diamètre interne du tube creux (15), la première portion creuse (34) s'encastrant en ajustement serré à l'intérieur du tube creux (15) et s'engageant à demeure dans le tube creux (15), et une seconde portion (32) avec une section de base ayant un diamètre extérieur sensiblement identique au diamètre extérieur du tube creux (15) et un alésage correspondant au diamètre interne de la première portion creuse (34) de la manille (30) et une paire d'éléments de bras opposés, espacés entre eux, parallèles (36) présentant des premières et secondes extrémités respectives, et assemblés solidairement sur la portion de base de la manille (30) respectivement sur les premières extrémités des éléments de bras opposés (36), la paire d'éléments de bras (36) s'étendant en éloignement de la première portion creuse de la manille (30), chaque élément de bras opposé (36) présentant une gorge en contre-dépouille (380) le long d'une surface extérieure de l'élément de bras (36) et sensiblement transversale à l'axe longitudinal, le revêtement plastique thermorétractable (20) aboutissant dans les gorges de contre-dépouille (380) des éléments de bras (36).

13. Instrument chirurgical selon la revendication 11 ou la revendication 12, comprenant de plus :
une virole (910) destinée à entourer et recouvrir une portion terminale du revêtement plastique thermorétractable (20) et une portion contigue du moyen d'actionnement (50).

14. Instrument chirurgical selon la revendication 13, dans lequel :
le moyen d'actionnement (50) comprend un logement (914) s'engageant à demeure dans le tube d'aluminium (15), dans lequel la virole (910) entoure et recouvre au moins une portion du logement (914) et un levier (420) coopère solidement avec la tige de poussée (60) et s'engage de façon pivotante dans le logement (914), de sorte que lors d'un mouvement pivotant du levier (420), un mouvement de va-et-vient est transmis à la tige de poussée (60).

15. Instrument chirurgical selon la revendication 14, dans lequel :
le moyen d'actionnement (50) comprend de plus une poignée (456) fixée à demeure sur le logement (914) pour coopérer avec le levier (420) et assurer un mouvement pivotant au levier (420) lorsque la poignée (456) et le levier (420) subissent une action de pincement par la main humaine.

16. Instrument chirurgical selon l'une quelconque des revendications 1 à 12, dans lequel :
le moyen d'actionnement (50) comprend un logement (914) s'engageant à demeure dans le tube extérieur (15), un levier (420) coopérant solidement avec la tige de poussée (60) et s'engageant de façon pivotante dans le logement (914) et une poignée (456) fixée à demeure sur le logement (914), de sorte que le mouvement pivotant du levier (420) transmet un mouvement de va-et-vient à la tige de poussée (60) et de sorte que la poignée (456) coopère avec le levier (420) pour assurer un mouvement pivotant au levier (420) lorsque la poignée (456) et le levier (420) subissent une action de pincement par la main humaine.

17. Instrument chirurgical selon l'une quelconque des revendications 9 à 16, dans lequel :
la première extrémité de la tige (60) présente une portion en forme de plaquette (122) avec le troisième trou (120) et le quatrième trou (120) espacé du troisième trou (120),
chacun des effecteurs d'extrémité (40) présente un premier trou respectif (39) pour recevoir les premiers moyens de rotation (45) et pour s'engager de façon pivotante avec la manille (30) au niveau des moyens de rotation (45) et un second trou respectif (98) sensiblement parallèle au premier trou, et
l'instrument chirurgical comprend un second élément en forme d'agrafe élastique unitaire (112) reliant la première extrémité de la tige (60) à l'un des effecteurs d'extrémité (40), avec l'un des moyens de connexion en forme d'agrafe élastique unitaire (110) s'engageant dans le troisième trou (120) de la tige et l'autre des éléments en forme d'agrafe élastique unitaire (112) s'engageant dans le quatrième trou (120) de la tige.

18. Instrument chirurgical selon la revendication 17, dans lequel :
les premier et second éléments (110, 112) qui s'engagent dans les trous traversants respectifs (120) de la portion en forme de plaquette (122) de la tige de poussée (60) sont positionnés de façon à s'entrecroiser.
